# EUROPÄISCHE PATENTSCHRIFT

(11) **EP 3 092 948 B1**
(45) Veröffentlichungstag und Bekanntmachung des Hinweises auf die Patenterteilung: **10.07.2019**
(21) Anmeldenummer: 16168105.1
(22) Anmeldetag: 03.05.2016
(51) Int. Cl.: A61B 5/107, A61B 5/00

(54) **METHODE ZUM ERKENNEN VON VERSCHMUTZUNGEN**
METHOD FOR DETECTING SOILING
PROCEDE DE RECONNAISSANCE D'IMPURETES

(30) Priorität: 07.05.2015 DE 102015107132
(43) Veröffentlichungstag der Anmeldung: 16.11.2016
(73) Patentinhaber: a.tron3d GmbH, 9020 Klagenfurt am Wörthersee (AT)
(72) Erfinder: Jesenko, Jürgen, 9584 Finkenstein (AT); Blassnig, Andreas, 9020 Klagenfurt (AT)
(74) Vertreter: Beer & Partner Patentanwälte KG

(56) Entgegenhaltungen:
- WO-A1-2013/010910
- DE-A1- 19 652 441
- DE-A1- 19 800 968
- DE-A1-102009 016 563
- US-A1- 2012 113 410

## Beschreibung

Die Erfindung betrifft ein Verfahren zum Betreiben einer Vorrichtung zum Erfassen der dreidimensionalen Geometrie von Objekten, insbesondere Zähnen, wobei die Vorrichtung wenigstens eine erste Kamera und eine transparente Schutzschicht zwischen Kamera und Objekt aufweist, wobei mit der Kamera wenigstens ein zweidimensionales Bild von wenigstens einem Teilbereich des Objektes aufgenommen wird, aus welchem dann eine mehrdimensionale Tiefeninformation des aufgenommenen Teilbereiches des Objektes erstellt wird und wobei verschiedene mehrdimensionale Tiefeninformationen von Teilbereichen zusammengeführt werden.

Im Bereich der Systeme zum optischen Erfassen von Oberflächengeometrien, insbesondere im Bereich der optischen intraoralen Scanner, sind inzwischen viele verschiedene Technologien bekannt. Dabei können die Scanner sowohl konstruktiv die unterschiedlichsten Formen annehmen als auch beim Erfassen der Oberflächengeometrien die verschiedensten Techniken/Verfahren anwenden. So zeigt die WO-2013/010910-A1 die Erfassung von dreidimensionalen Geometrien. DE-19652441-A1, US-2012/113410-A1, DE-19800968-A1, und DE-102009016563-A1 zeigen die Erkennung von Verschmutzungen.

Allen Systemen gemeinsam sind jedoch zwei wesentliche Eigenschaften: Zum einen benötigen alle einen Bildsensor, eine Kamera oder dergleichen, um damit zweidimensionale Bilder des Objektes oder von Teilbereichen des Objektes aufzunehmen. Zum anderen wird bei allen optischen Methoden ein Abstand zwischen einem realen oder virtuellen Element der Vorrichtung und dem Objekt ermittelt. Dieser Abstand wird üblicherweise je Bildpunkt des zweidimensionalen Bildes erfasst und in vielen Fällen notiert. Dabei gibt es zwar zwingend für jeden ermittelten Abstand einen korrespondierenden Bildpunkt, allerdings kann nicht immer zu jedem Bildpunkt ein Abstand ermittelt werden. Eine solche "Karte" aus Pixelkoordinaten bzw. Bildpunkten und Abstandswerten wird vom Fachmann üblicherweise als Tiefenbild bezeichnet. Alternativ kann aus den Abständen auch eine Punktwolke erzeugt werden. Da in beiden Fällen allerdings nur eine Oberflächenstruktur ermittelt wird, diese aber noch in keinem echten dreidimensionalen Kontext steht, werden solche Tiefenbilder und Punktwolken auch häufig als zweieinhalbdimensional oder 2,5D bezeichnet. So wird ihre zwischen zweidimensionalen Aufnahmen und echtem 3D liegende Natur repräsentiert. Sowohl die Methoden, um von einer oder mehreren zweidimensionalen Aufnahmen zu solchen mehrdimensionalen Tiefeninformationen zu gelangen, als auch die Methoden, um mehrere mehrdimensionale Tiefeninformationen zu einer dreidimensionalen Repräsentation des Objektes zusammen zu führen, können stark voneinander abweichen.

Neben den bereits genannten Gemeinsamkeiten im physischen Aufbau von Vorrichtungen zum optischen Erfassen von Oberflächengeometrien von Objekten haben Intraoral-Scanner eine weitere Gemeinsamkeit, welche sich aus ihrem speziellen Anwendungsgebiet ergibt. Da Intraoral-Scanner im Wesentlichen zum Scannen von Zähnen verwendet werden, haben diese in der Regel eine weitere physische Gemeinsamkeit, nämlich einen Kopfbereich, welcher in seinen Ausmaßen geeignet sein muss, in den Mund eines Patienten eingeführt zu werden und gegebenenfalls hinreichend Bewegungsspielraum zu haben, um alle gewünschten Zähne aus allen benötigten Perspektiven erfassen zu können. Dieser Kopfbereich kann von einfachen Umlenkspiegeln bis hin zu vollständigen optischen Systemen mit einer oder mehreren Kameras und/oder Projektoren verschiedenste Elemente enthalten.

Unabhängig von den darin angeordneten Elementen hat es sich als zweckmäßig herausgestellt, den Kopfbereich mit einer Verkleidung zu versehen. Zum einen sind dadurch die - zum Teil sehr empfindlichen - elektronischen Bauteile des Scanners vor Feuchtigkeit geschützt, welche beispielsweise mit der Atemluft transportiert wird. Zum anderen kann der Teil des Scanners, welcher in Kontakt mit Patienten kommen kann, leicht desinfiziert werden.

Es versteht sich von selbst, dass wenigstens ein Teil der Umhüllung transparent gestaltet werden muss, um die optische Vermessung des Objektes zu ermöglichen. Allerdings kommt es häufig vor, dass eine solche transparente Schutzschicht zwischen Objekt und Kamera bzw. Bildsensor während des Betriebs beispielsweise durch Speichel oder Blut verschmutzt wird. Dadurch wird das Erfassen der dreidimensionalen Geometrien verfälscht und die Daten können nicht mehr in der für dentale Anwendungen benötigten Präzision erfasst werden.

Wenn diese Verschmutzungen durch eine Bedienungsperson bemerkt werden, ist die erfasste dreidimensionale Oberflächengeometrie häufig bereits so stark verfälscht, dass es sinnvoll ist, einen neuen Scan zu starten. Dies ist sowohl für die Bedienungsperson als auch für den Patienten unangenehm und, abhängig von den Betriebskosten des Scanners, gegebenenfalls auch teuer, und soll daher vermieden werden.

Der Erfindung liegt daher die Aufgabe zu Grunde, die oben beschriebenen Nachteile zu überwinden, indem eine Verschmutzung der transparenten Schutzschicht frühzeitig automatisch erkannt wird.

Gelöst wird diese Aufgabe erfindungsgemäß durch ein Verfahren nach Anspruch 1.

Die Meldung kann beispielsweise ein Symbol auf einem Display sein, eine an einem Handstück, an welchem sich der Kopfbereich befindet, aufleuchtende LED, ein Geräusch oder auch ein Vibrieren des Handstücks.

Das Element, zu welchem die Abstände notiert werden, kann dabei auch ein rein virtuelles Element sein. Wenn beispielsweise der Abstand zur Kamera ermittelt wird und beispielsweise aus ergonomischen oder platztechnischen Gründen Umlenkspiegel im Kopfbereich oder einem anderen Bereich des Handstücks angeordnet sind, dann kann der Abstand zu einem virtuellen Punkt im Raum notiert werden, an dem sich die Kamera befände, wenn der Sichtkegel der Kamera nicht umgelenkt werden würde. Alternativ kann für die Zwecke der Notation aber auch ein beliebiger anderer Punkt gewählt werden. Der Abstand zur Kamera würde in diesem Beispiel dann um ein gleichbleibendes Offset verringert oder gegebenenfalls auch vergrößert werden. Da auch ein derartiger virtueller Raumpunkt in Bezug zur Vorrichtung steht, wäre dieser gedachte Raumpunkt - trotzdem er kein physisches Element der Vorrichtung ist - dennoch ein Element der Vorrichtung im Sinne der Erfindung.

Die beiden gängigsten Methoden, um zu mehrdimensionalen Oberflächeninformationen, insbesondere 2,5D-Daten, zu gelangen, sind die "structured light" Methode und die Stereometrie.

Bei der Stereometrie werden mit zwei Kameras, deren räumliche Relation und Ausrichtung zueinander bekannt ist, zwei zweidimensionale Bilder, vorzugsweise simultan, aufgenommen. Zwei derart aufgenommene Bilder werden auch Stereobildpaar genannt. Dann werden die aus dem (geringfügig) unterschiedlichen Blickwinkel der Kameras resultierenden Unterschiede in dem Stereobildpaar (Disparitäten) ermittelt und vermessen. Dabei wird der Abstand zu einer gedachten Verbindungslinie zwischen beiden Kameras, der so genannten Stereobasis, ermittelt. Daher ist das Element der Vorrichtung bevorzugt eine Stereobasis, welche zwischen der ersten Kamera und einer zweiten Kamera gebildet wird.

Bei der "structured light" Methode wird ein bekanntes Muster mit einem Projektor auf das Objekt projiziert, welches durch die Kamera aufgenommen wird. Dann werden Verzerrungen des Musters, welche durch die Oberflächenstruktur des Objektes erzeugt werden, im zweidimensionalen Bild analysiert und es werden Tiefeninformationen gewonnen. Dabei wird ähnlich wie bei der Stereometrie das bekannte räumliche Verhältnis von Projektor und Kamera zueinander hinzugezogen. Auch bei dieser Methode wird üblicherweise eine gedachte Verbindungslinie zwischen Projektor und Kamera für die Berechnung der Abstände verwendet. Diese Linie nennt man ebenfalls Stereobasis. Daher ist das Element der Vorrichtung bevorzugt eine Stereobasis, welche zwischen der ersten Kamera und einem Projektor gebildet wird.

Auch wenn diese beiden Methoden häufig alternativ zueinander verwendet werden, sind auch Kombinationen von beiden denkbar. So kann beispielsweise auch ein Muster auf das Objekt projiziert werden, um stereometrische Aufnahmen zu unterstützen. Dabei hilft das Muster, mehr Punkte in beiden Bildern des Stereobildpaares zu identifizieren, die dann verglichen werden können, um Disparitäten in den Bildern des Stereobildpaares zu ermitteln. Da für eine stereometrische Vermessung beide Kameras "Sicht" auf eine zu vermessende Stelle des Objektes haben müssen, kann es vorkommen, dass stereometrische Aufnahmen an besonders engen Stellen, beispielsweise im Bereich der Molaren, nicht in ausreichender Qualität möglich sind. Ist das Verhältnis des in einem solchen Aufbau in erster Linie unterstützend arbeitenden Projektors zu den Kameras bekannt, kann dieser an besonders unzugänglichen Stellen, die nur durch eine der Kameras erfasst werden können, verwendet werden, um Tiefenbilder mittels der "structured light" Methode zu ermitteln.

Der definierte Wert wird dabei so gewählt, dass alle Abstände, welche sich direkt an der transparenten Schutzschicht befinden, unter diesem definierten Wert liegen.

Der definierte Prozentsatz kann dabei von verschiedenen Faktoren abhängen. Sind an einem Gehäuse des Scanners Abstandhalter vorgesehen, ist ein direkter Kontakt von Objekt und transparenter Schicht sehr selten oder kommt gar nicht vor. Entsprechend kann der Prozentsatz, bei welchem eine Meldung ausgelöst wird, sehr gering gewählt werden. Handelt es sich hingegen um einen Scanner, bei welchem vorgesehen ist, dass die transparente Schicht auf dem Objekt aufliegt oder aufliegen kann, wird automatisch ein gewisser Prozentsatz der ermittelten Abstände den definierten Wert unterschreiten. In diesem Fall ist es vorteilhaft, wenn der Prozentsatz entsprechend hoch definiert wird.

In einer Weiterbildung der Erfindung kann auch im Zuge eines Kalibrierverfahrens ein statistisch zu erwartender Prozentsatz für Abstände unterhalb des definierten Wertes ermittelt werden. Der definierte Prozentsatz wird dann einfach geringfügig über dem zu erwartenden Prozentsatz festgelegt.

Eine mögliche, beispielhafte Ausführungsform dieser Weiterbildung wäre folgendes einfaches Verfahren:
a) Ein Zahnmodell, beispielsweise aus Gips, bei welchem entsprechend keine Speichel- oder Blutspritzer die Messung verfälschen können, wird gescannt.
   Idealerweise ist dieses in einer Nachbildung einer Mundhöhle untergebracht, um den eingeschränkten Bewegungsfreiraum, welchem der Scanner in der Realität unterliegt, ebenfalls zu berücksichtigen.
b) Die dabei entstehenden mehrdimensionalen Tiefeninformationen werden gesammelt und auf die durchschnittliche und höchste Anzahl an Abständen unterhalb des definierten Wertes pro Tiefenbild untersucht.
   Anders als bei einem normalen Scanvorgang, in welchem die mehrdimensionalen Tiefeninformationen in einem weiteren Schritt zu einem gesamten 3D-Modell zusammengeführt werden, ist hierfür keine weitere Verarbeitung bzw. Überführung der Tiefenbilder notwendig.
c) Aus der durchschnittlichen und der höchsten Anzahl von Abständen unterhalb des definierten Wertes pro mehrdimensionaler Tiefeninformation und der durchschnittlichen und gegebenenfalls auch der höchsten Anzahl von Abständen pro mehrdimensionaler Tiefeninformatione allgemein wird dann der Prozentsatz ermittelt, ab welchem von einer Verschmutzung der transparenten Schutzschicht auszugehen ist.

In einer Weiterbildung der Erfindung können der durchschnittliche Anteil an Abständen unterhalb des definierten Wertes und der höchste Anteil an Abständen unterhalb des definierten Wertes auch separat genutzt werden. So kann eine erste Art von Meldung gegeben werden, wenn der durchschnittliche Prozentsatz überschritten wird und eine zweite Art von Meldung gegeben werden, wenn der höchste Prozentsatz überschritten wird. Bei der zweiten Art von Meldung kann auch eine Unterbrechung des Scanvorganges ausgelöst werden. Beispielsweise könnte die LED am Handstück bei der ersten Art von Meldung zunächst gelb und bei der zweiten Art von Meldung dann rot aufleuchten. Um die Wahrnehmung besonders intuitiv zu gestalten, kann die LED im normalen Betrieb zusätzlich beispielsweise in grün oder blau leuchten. Die Meldungen entsprechen in diesem Ausführungsbeispiel dann den Farbwechseln.

Da ein Verfälschen der Messwerte generell unerwünscht ist und dies je nach der gewählten Methode, mit welcher die mehrdimensionalen Tiefeninformationen in ein 3D-Modell überführt werden, nur schwer wieder zu korrigieren ist, kann ein Unterbrechen des Scanvorgangs beim Erreichen des definierten Prozentsatzes wünschenswert sein. In einer bevorzugten Durchführungsform des Verfahrens wir daher durch das Auslösen der Meldung das Erfassen der dreidimensionalen Geometrie angehalten.

Weitere bevorzugte Ausführungsformen der Erfindung sind Gegenstand der übrigen Unteransprüche.

Nachstehend ist ein bevorzugtes Ausführungsbeispiel der Erfindung anhand der Zeichnungen näher beschrieben. Es zeigt:
- Fig. 1: schematisiert dargestellt eine Vorrichtung zum Erfassen der dreidimensionalen Geometrie von Objekten, insbesondere Zähnen, mit symbolisch dargestellten Messwerten,
- Fig. 2: die symbolisch dargestellten Messwerte von Fig. 1 und
- Fig. 3: einen beispielhaften Ablauf eines erfindungsgemäßen Verfahrens.

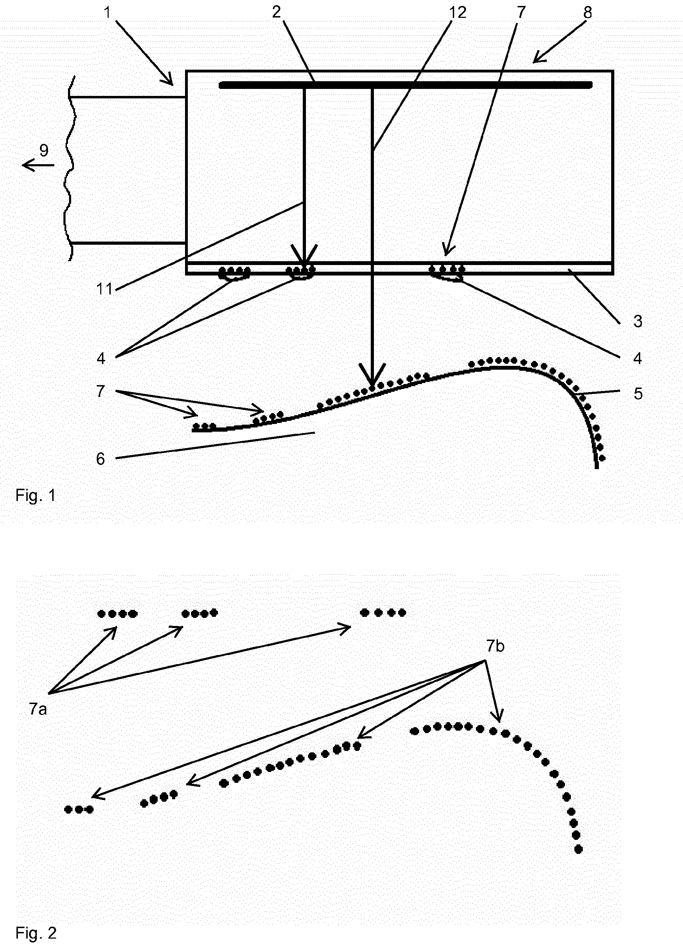

Die Fig. 1 zeigt schematisiert dargestellt eine Vorrichtung 1 zum Erfassen der dreidimensionalen Geometrie von Objekten, insbesondere Zähnen, mit einem Bildsensor 2 und einer transparenten Schutzschicht 3 in Form eines Abdeckglases mit Verschmutzungen 4, einen Teilbereich 5 eines Objektes 6 und in Form von Punkten 7 symbolisch dargestellte, gemessene Oberflächenpunkte. In der beispielhaften und stark schematisierten Darstellung von Fig. 1 handelt es sich bei dem Element der Vorrichtung 1 um den Bildsensor 2. Zur Veranschaulichung zeigt die Darstellung lediglich zwei Dimensionen, der in der Realität dreidimensionalen Oberfläche des Objektes 6. In der Realität kann eine Vorrichtung 1 zum Erfassen der dreidimensionalen Geometrie von Objekten verschiedene optische Elemente, wie Linsen, Blenden, Spiegel, Dias, Strahlteiler, Lichtquellen, Projektoren und dergleichen enthalten. Diese sind aus Gründen der Übersicht nicht dargestellt. Weiters ist von der Vorrichtung 1 lediglich ein Kopfbereich 8 gezeigt, der in einen Griffbereich 9 übergeht. Peripheriegeräte, wie Stromversorgung, eine Recheneinheit oder dergleichen sind ebenfalls nicht dargestellt. Hierzu sind hinreichend viele Varianten und Möglichkeiten aus dem Stand der Technik bekannt und können entsprechend vom Fachmann gewählt werden.

Ein optisch arbeitender Intraoral-Scanner vermisst lediglich Oberflächen, die von diesem auch "gesehen" werden. Dies führt unter anderem dazu, dass es notwendig ist, ein Objekt von verschiedenen Seiten zu betrachten, um es vollständig zu erfassen. Weiters müssen diese unterschiedlichen "Ansichten" in einem weiteren Schritt zusammengefügt werden, um ein idealerweise vollständiges, virtuelles 3D-Modell des Objektes zu erhalten.

Fig. 1 illustriert, wie Verschmutzungen 4 die Abstandsmessung verfälschen. Ein erster Abstand, symbolisch darstellt durch einen ersten Pfeil 11, der in dem Bereich gemessen wird, in dem die Verschmutzung 4 das Abdeckglas 3 bedeckt, ist zu kurz und gibt nicht den Abstand vom Bildsensor 2 zum Objekt 6 wieder. Demgegenüber zeigt ein zweiter Pfeil 12 einen korrekt vermessenen zweiten Abstand vom Bildsensor 2 zum Objekt 6 wieder. Die Punkte 7 sind dabei symbolisch für weitere Abstandsmessungen eingezeichnet. In der Realität kann es sowohl vorkommen, dass die Abstandwerte in einer wesentlich größeren Dichte erfasst werden, als auch, dass für einige Bereiche überhaupt keine Abstandswerte erfasst werden oder die Abstandswerte unterschiedlich dicht erfasst werden.

Die Messung des Abstandes zwischen Objekt und Bildsensor ist dabei eine im Sinne der Veranschaulichung gewählte, starke Simplifizierung und kommt in dieser Form in der Realität kaum vor. Die tatsächlich verwendeten virtuellen und/oder physischen Bezugspunkte für diese Abstandsmessungen sind jedoch aus dem Stand der Technik hinreichend bekannt und die gegenständliche Erfindung kann vom Fachmann auf die verschiedenen bekannten Systeme ohne weiteres übertragen werden.

Die Fig. 2 zeigt die symbolisch dargestellten Messwerte von Fig. 1 ohne die Vorrichtung 1 und den Teilbereich 5 des Objektes 6. Dabei stellen die Punkte 7a jene Punkte dar, die - mit den zu erwartenden Schwankungen und Messfehlern - korrekt gemessen wurden. Die Punkte 7b stellen dagegen Messungen dar, die aufgrund der Verschmutzungen erfolgt sind und nicht in Bezug zu der zu vermessenden Oberfläche stehen. Die Punkte 7b und die Punkte 7a sind dabei für das System zunächst nicht zu unterscheiden. Wird allerdings wie erfindungsgemäß vorgesehen ein Abstandswert definiert, der - im Rahmen vorhersehbarer Schwankungen - dem Abstand der transparenten Schutzschicht 3 zum Element 2 entspricht, kann das System die gemessenen Abstandswerte in Werte unter und über einem definierten Wert unterteilen.

Grundsätzlich ist es möglich, jeden Wert, der entsprechend als "zu nah" eingestuft wird, als Fehler, der von Verschmutzungen verursacht wurde, zu interpretieren. Da der Scanner 1 mit der transparenten Schutzschicht 3 aber auch direkt auf dem zu vermessenden Objekt zu liegen kommen kann, ist es sinnvoll, zunächst den Anteil an Messungen, welche als an der transparenten Abdeckung 3 befindlich (also den definierten Wert unterschreitend) identifiziert wurden, zu ermitteln und erst bei Überschreiten eines definierten Prozentsatzes durch das System eine Verschmutzung festzustellen. Sollte keine Berührung der Schutzschicht erwünscht sein, kann der definierte Prozentsatz auch auf 0% gesetzt werden.

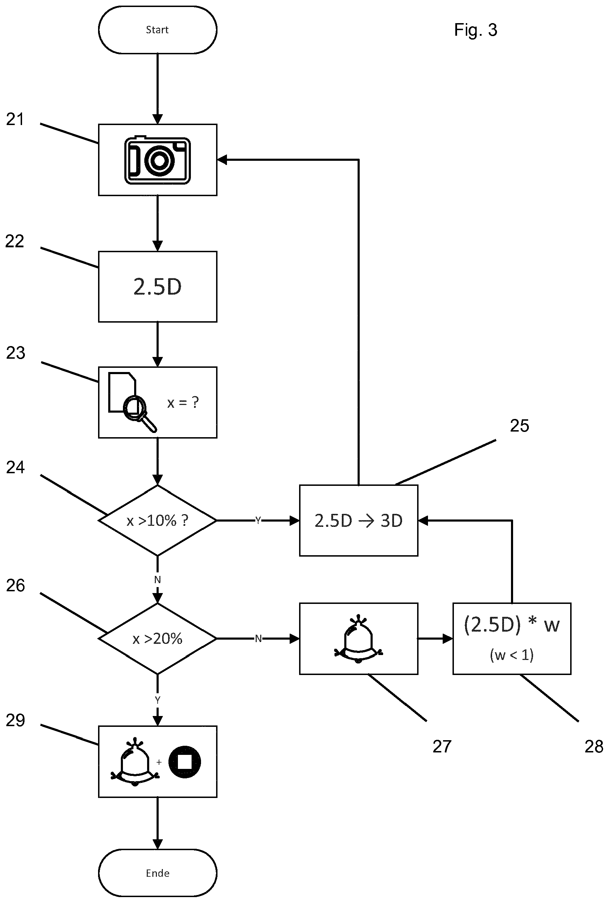

Einen beispielhaften Ablauf einer bevorzugten Ausführungsform des erfindungsgemäßen Verfahrens zeigt die Fig. 3.

Dabei wird zunächst in einem ersten Schritt 21 wenigstens ein zweidimensionales Bild des zu erfassenden Objektes aufgenommen.

Dieses wird dann in einem zweiten Schritt 22 in eine mehrdimensionale Tiefeninformation, im dargestellten Fall ein 2,5D Tiefenbild, umgewandelt. Dabei werden Abstandswerte zu einem Element der Vorrichtung ermittelt. In der beispielhaften und stark schematisierten Darstellung von Fig. 1 handelt es sich bei dem Element der Vorrichtung 1 um den Bildsensor 2.

Wie bereits weiter oben erläutert, ist es in der Realität aber häufig zielführender, andere reale oder virtuelle Elemente der Vorrichtung 1 für die Abstandsmessung zu verwenden. Sowohl das Bezugselement für die Abstandsmessung als auch der Wert, ab dem Messungen als "zu nah" eingestuft werden, können vom Fachmann entsprechend der verwendeten Messmethode und dem physischen Aufbau des Scanners gewählt werden.

In einem dritten Schritt 23 werden die erfassten Abstände dann daraufhin untersucht, wie hoch ein Anteil x der Messwerte ist, der unter dem definierten Wert liegt. In einem vierten Schritt 24 wird abgefragt, ob der Anteil der Abstände unter dem definierten Wert unter einem ersten Prozentsatz liegt. Im dargestellten Beispiel ist für den ersten Prozentsatz 10% festgesetzt. Der definierte Prozentsatz kann abhängig vom System und den jeweiligen Anforderungen selbstverständlich auch auf einen anderen Wert festgesetzt werden. Wenn der Prozentsatz unter dem definierten Wert liegt, wird das 2,5D Tiefenbild in einem fünften Schritt 25 in ein globales 3D-Modell eingefügt und es werden weitere Aufnahmen gemacht. Ist der Prozentsatz höher, kann, wie im Beispiel in einem sechsten Schritt 26 dargestellt, eine weitere Abfrage mit einem zweiten definierten Prozentsatz erfolgen. Auch dieser kann frei gewählt werden; allerding ist es sinnvoll, diesen über dem ersten definierten Prozentsatz zu wählen. Liegt der ermittelte Anteil unter dem zweiten definierten Prozentsatz, wird in einem siebten Schritt 27 eine Meldung gegeben. Diese kann beispielsweise akustisch und/oder visuell erfolgen.

Weiters kann dem 2,5D Tiefenbild, respektive den darin enthaltenen Werten, in einem achten Schritt 28 ein Gewicht w gegeben, welches eine Gewichtung der Werte im 3D-Modell verringert. So können die Werte zwar noch immer in das 3D-Modell einfließen. Sollten sie aber bereits aufgrund von Verschmutzungen fehlerhaft sein, können sie leichter wieder durch korrekte Werte korrigiert werden. Details zum Gewichten von Messwerten im Zusammenhang mit dem Erfassen von Oberflächengeometrien, insbesondere im Intraoral-Bereich, und mögliche Implementierungen können beispielsweise der US 2015/0024337 A1 entnommen und beim gegenständlichen Verfahren angewandt werden.

Die Schritte siebter Schritt 27 und achter Schritt 28 können in beliebiger Reihenfolge oder auch simultan erfolgen. Liegt der Anteil auch über dem zweiten definierten Prozentsatz, ist anzunehmen, dass die transparente Schutzschicht 3 verschmutzt ist und dass weitere Messungen das 3D-Modell verfälschen. Es wird daher in einem neunten Schritt 29 eine Meldung an den Nutzer gegeben, beispielsweise eine Aufforderung, die transparente Schutzschicht 3 zu reinigen. Gleichzeitig wird das Erfassen angehalten, um so das Verfälschen des 3D-Modells zu verhindern.

## Patentansprüche

1. Verfahren zum Betreiben einer Vorrichtung zum Erfassen der dreidimensionalen Geometrie von Objekten, insbesondere Zähnen, wobei die Vorrichtung wenigstens eine erste Kamera und eine transparente Schutzschicht zwischen Kamera und Objekt aufweist, und zur Erkennung einer Verschmutzung der transparenten Schutzschicht,
wobei mit der Kamera wenigstens ein zweidimensionales Bild von wenigstens einem Teilbereich des Objektes aufgenommen wird, aus welchem dann eine mehrdimensionale Tiefeninformation des aufgenommenen Teilbereiches des Objektes erstellt wird und wobei verschiedene mehrdimensionale Tiefeninformationen von Teilbereichen zusammengeführt werden,
wobei zu den mehrdimensionalen Tiefeninformationen eine Kombination von Pixelkoordinaten des zweidimensionalen Bildes und jeweils einer Pixelkoordinate zugeordneten Abständen zu einem Element der Vorrichtung notiert wird und wobei, wenn die Abstände zu einem definierten Prozentsatz einen definierten Wert unterschreiten, eine Meldung ausgelöst wird.

2. Verfahren nach Anspruch 1, **dadurch gekennzeichnet, dass** das Element der Vorrichtung eine Stereobasis ist, welche zwischen der ersten Kamera und einem Projektor gebildet wird.

3. Verfahren nach Anspruch 1, **dadurch gekennzeichnet, dass** das Element der Vorrichtung eine Stereobasis ist, welche zwischen der ersten Kamera und einer zweiten Kamera gebildet wird.

4. Verfahren nach Anspruch 1, **dadurch gekennzeichnet, dass** das Element ein Projektionszentrum eines Stereosystems der ersten und der zweiten Kamera oder einer Kamera und des Projektors ist.

5. Verfahren nach einem der Ansprüche 1 bis 4, **dadurch gekennzeichnet, dass** der definierte Abstand durch den Abstand zwischen der Stereobasis und einer Außenseite der transparenten Schutzschicht definiert wird.

6. Verfahren nach einem der Ansprüche 1 bis 5, **dadurch gekennzeichnet, dass** durch das Auslösen der Meldung das Erfassen der dreidimensionalen Geometrie angehalten wird.

7. Verfahren nach einem der Ansprüche 1 bis 6, **dadurch gekennzeichnet, dass** der definierte Prozentsatz zwischen 20% und 5% liegt, vorzugsweise 10% beträgt.

8. Verfahren nach einem der Ansprüche 1 bis 7, **dadurch gekennzeichnet, dass** die mehrdimensionalen Tiefeninformationen Tiefenbilder sind.

9. Verfahren nach einem der Ansprüche 1 bis 7, **dadurch gekennzeichnet, dass** die mehrdimensionalen Tiefeninformationen Punktwolken sind.

## Claims

1. A method for operating a device for detecting the three-dimensional geometry of objects, in particular teeth, wherein the device comprises at least a first camera and a transparent protective layer between camera and object, and for detecting a contamination of the transparent protective layer,
wherein the camera is used to capture at least one two-dimensional image of at least one partial region of the object, which is then used to generate multi-dimensional depth data of the captured partial region of the object and wherein different multi-dimensional depth data from partial regions is combined,
wherein a combination of pixel coordinates of the two-dimensional image and distances associated with respectively one pixel coordinate from one element of the device is recorded with regard to the multidimensional depth data and wherein a message is triggered if the distances for a defined percentage drop below a defined value.

2. The method according to claim 1, **characterised in that** the element of the device is a stereo base, which is formed between the first camera and a projector.

3. The method according to claim 1, **characterised in that** the element of the device is a stereo base, which is formed between the first camera and a second camera.

4. The method according to claim 1, **characterised in that** the element is a projection centre of a stereo system of the first camera and the second camera or a camera and the projector.

5. The method according to one of claims 1 to 4, **characterised in that** the defined distance is defined by the distance between the stereo base and an outer side of the transparent protective layer.

6. The method according to one of claims 1 to 5, **characterised in that** detection of the three-dimensional geometry is stopped by triggering the message.

7. The method according to one of claims 1 to 6, **characterised in that** the defined percentage lies between 20% and 5%, preferably at 10%.

8. The method according to one of claims 1 to 7, **characterised in that** the multi-dimensional depth data represents depth images.

9. The method according to one of claims 1 to 7, **characterised in that** the multi-dimensional depth data represents point clouds.

## Revendications

1. Procédé pour exploitation d'un dispositif pour la détection de la géométrie tridimensionnelle d'objets, en particulier de dents, dans lequel le dispositif présente au moins une première caméra et une couche de protection transparente entre la caméra et l'objet, et pour la reconnaissance d'un encrassement de la couche de protection transparente, dans lequel au moins une image bidimensionnelle d'au moins une zone partielle de l'objet est enregistrée avec la caméra, à partir de laquelle une information de profondeur multidimensionnelle de la zone partielle de l'objet enregistrée est établie, et dans lequel différentes informations de profondeur multidimensionnelles de zones partielles sont groupées, dans lequel on note, pour les informations de profondeur multidimensionnelles, une combinaison de coordonnées de pixels de l'image bidimensionnelle et de distances par rapport à un élément du dispositif respectivement associées à une coordonnée de pixel, et dans lequel, si les distances tombent au-dessous une valeur définie de l'ordre d'un pourcentage défini, un message est déclenché.

2. Procédé selon la revendication 1, **caractérisé en ce que** l'élément du dispositif est une base stéréo formée entre la première caméra et un projecteur.

3. Procédé selon la revendication 1, **caractérisé en ce que** l'élément du dispositif est une base stéréo formée entre la première caméra et une deuxième caméra.

4. Procédé selon la revendication 1, **caractérisé en ce que** l'élément est un centre de projection d'un système stéréo de la première et la deuxième caméra ou d'une caméra et du projecteur.

5. Procédé selon l'une des revendications 1 à 4, **caractérisé en ce que** la distance définie est définie par la distance entre la base stéréo et un côté extérieur de la couche de protection transparente.

6. Procédé selon l'une des revendications 1 à 5, **caractérisé en ce que**, par le déclenchement du message, la détection de la géométrie tridimensionnelle est arrêtée.

7. Procédé selon l'une des revendications 1 à 6, **caractérisé en ce que** le pourcentage défini est compris entre 20% et 5%, et est de préférence de 10%.

8. Procédé selon l'une des revendications 1 à 7, **caractérisé en ce que** les informations de profondeur multidimensionnelles sont des images de profondeur.

9. Procédé selon l'une des revendications 1 à 7, **caractérisé en ce que** les informations de profondeur multidimensionnelles sont des nuages de points.
